(19) Europäisches Patentamt / European Patent Office / Office européen des brevets

(11) **EP 4 081 293 B1**

(12) **EUROPEAN PATENT SPECIFICATION**

(45) Date of publication and mention
of the grant of the patent:
**08.10.2025 Bulletin 2025/41**

(21) Application number: **20842730.2**

(22) Date of filing: **23.12.2020**

(51) International Patent Classification (IPC):
*A61N 7/02* [(2006.01)]   *A61B 8/08* [(2006.01)]
*G06T 7/11* [(2017.01)]   *A61B 34/30* [(2016.01)]
*A61B 8/00* [(2006.01)]   *A61B 5/11* [(2006.01)]
*A61B 34/10* [(2016.01)]

(52) Cooperative Patent Classification (CPC):
**A61N 7/02; A61B 5/015; A61B 5/4887;**
**A61B 8/5207; A61B 8/5223; G01K 11/26;**
**G06T 7/0012;** A61B 5/1127; A61B 5/7257;
A61B 8/4245; A61B 34/10; A61B 34/30;
A61B 2034/104; A61B 2034/105; A61B 2034/2048;
(Cont.)

(86) International application number:
**PCT/IB2020/062408**

(87) International publication number:
**WO 2021/130702 (01.07.2021 Gazette 2021/26)**

(54) **DEVICE FOR MONITORING HIFU TREATMENTS**

VORRICHTUNG ZUR ÜBERWACHUNG VON HIFU-BEHANDLUNGEN

DISPOSITIF DE SURVEILLANCE DES TRAITEMENTS HIFU

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **23.12.2019 IT 201900025306**

(43) Date of publication of application:
**02.11.2022 Bulletin 2022/44**

(73) Proprietor: **Imedicals Srl**
**73100 Lecce (IT)**

(72) Inventor: **CASCIARO, Sergio**
**73100 Lecce (IT)**

(74) Representative: **Conversano, Gabriele**
**Laforgia, Bruni & Partners srl**
**Via Michele Garruba, 3**
**70122 Bari (IT)**

(56) References cited:
**US-A1- 2015 005 634   US-B2- 8 016 757**

• **ANAND A ET AL: "Noninvasive Determination of**
**in situ Heating Rate Using kHz Acoustic**
**Emissions and Focused Ultrasound",**
**ULTRASOUND IN MEDICINE AND BIOLOGY,**
**NEW YORK, NY, US, vol. 35, no. 10, 1 October**
**2009 (2009-10-01), pages 1662 - 1671,**
**XP026641061, ISSN: 0301-5629, [retrieved on**
**20090821], DOI: 10.1016/**
**J.ULTRASMEDBIO.2009.05.015**

(52) Cooperative Patent Classification (CPC): (Cont.)
A61B 2034/2051; A61B 2090/067; A61B 2090/378;
A61B 2090/3954; A61B 2562/0219;
A61N 2007/0052; A61N 2007/0073; G06N 3/08;
G06T 2207/10132; G06T 2207/30096

**Description**

Technical field

The present Patent application for industrial invention relates to a method for the treatment of a tumor area by means of high intensity focused ultrasound (HIFU - High Intensity Focused Ultrasound) combined with monitoring the temperature distribution and the actually treated area.

State of the art

There are many documents describing tumor mass treatments by means of high intensity focused ultrasound (HIFU).

During a HIFU treatment, one of the main problems is to determine the actual temperature spatial distribution in real time during the treatment.

An example of temperature monitoring by means of the analysis of the ultrasound signal reflected by tissues is described in US2015005634, where a method is described which uses the variation of acoustic attenuation in tissues while increasing temperature, and, on the basis of this effect, measures temperature by detecting temperature variation of the harmonics of the ultrasound signal reflected by tissues as a response to the high temperature pulses.

However, the method described requires a calibration on fictitious tissue portions (phantom) in order to quantify the variations in harmonic parameters as a function of temperature variations. It is clear that the phantoms used to study the problem (i) do not allow to consider the heat diffusive phenomena linked to body fluids circulation and, however (ii) approach only partially the real tissue features, since tissues of different body parts have different behavior and, in case of equal body part interested, the tissues of different subjects have a different behavior, in particular with reference to the actual acoustic heating.

Technical problem

Other embodiments of temperature non-invasive measuring methods are described in documents US2020237234, US2019142513, US2019262074, WO2019173138, but none of these ones solves the technical problem to measure a temperature spatial distribution by simply considering the specific features of tissues undergoing a HIFU treatment, all by means of a unique ultrasound probe and in real time. Further prior art is disclosed in US 8 016 757.

More generally, to the best knowledge of the current inventors, some problems linked to the usage of ultrasound signals for diagnosis and treatment of tumor masses in various organs of the human body remain unsolved.

In fact, embodiments which, by means of a unique ultrasound probe, allow: (i) to individuate the tumor mass; (ii) to verify the correct positioning of the probe with respect to the tumor mass; (iii) to treat the tumor mass by means of HIFU; (iv) to verify the extension of the area actually treated by temperature monitoring in real time are not known.

Aim of the invention

Therefore, aim of the present invention is to provide a method for tumor mass treatment, which, by means of a unique ultrasound probe, allows: (i) to individuate the tumor mass; (ii) to verify the correct positioning of the probe with respect to the tumor mass; (iii) to treat the tumor mass by means of HIFU; (iv) to verify the extension of the area actually treated by temperature monitoring in real time.

More in particular, aim of the present invention is to provide an ultrasound device and a method which allow to measure a temperature spatial distribution by simply considering the specific features of the tissues undergoing a HIFU treatment, and in particular the actual acoustic heating rate, all by using a unique ultrasound probe and in real time.

Detailed description of the invention

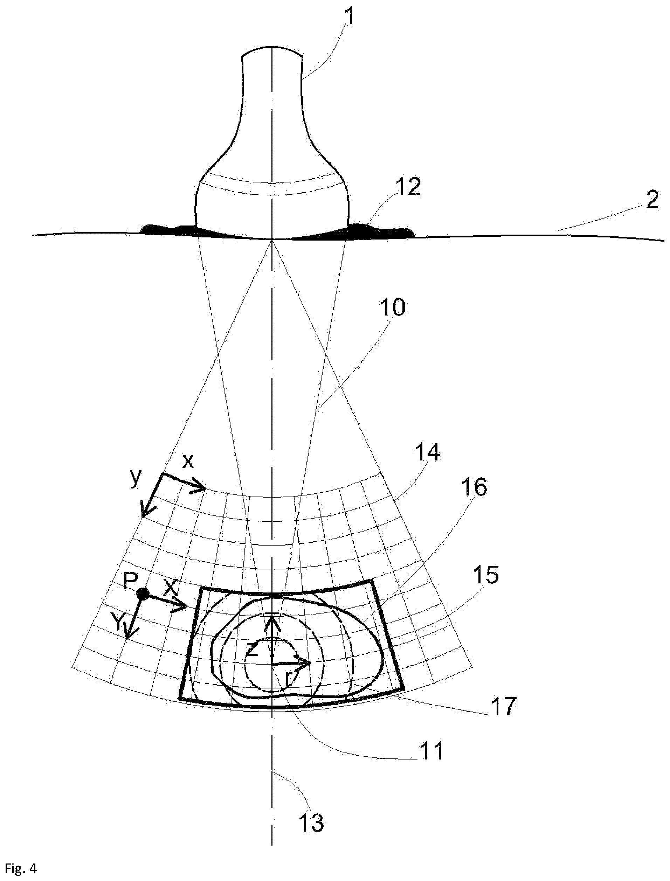

These and other advantages will be clear from the detailed description of the invention, which will refer to the appended drawings 1 to 6. In figure 1 it is shown an example image of an ultrasound probe (1) positioned on the abdomen of a patient (2), the positioning at the abdomen indicating only one application of the invention; in figure 2 it is shown a diagram of the temperature variation in time at the focal area after HIFU treatment and operating cycle of the device according to the invention; in figures 3 to 5 there are shown example schemes of the method of treatment by means of HIFU according to the invention; in figure 6-a it is shown a frequency spectrum of a tissue in which there are no localized boiling and/or cavitation phenomena; in figure 6-b it is shown a frequency spectrum of a tissue in which localized boiling and/or cavitation phenomena are in progress.

Firstly, it is to be said that in the context of the present Patent application, with "raw ultrasound signal" or "radiofrequency ultrasound signal" it is intended the ultrasound signal emitted by the probe and reflected by the human body towards the same probe, before the same is processed in order to obtain the ultrasound image; yet, if not otherwise specified, it is to be specified that with "ultrasound image" it is intended an ultrasound image of B-mode type, obtained along the propagation plane of the ultrasound beam emitted by the probe.

Yet, it is to be specified that with "raw ultrasound signal corresponding to a determined ROI" it is intended the portion of the raw ultrasound signal which, suitably treated, originated a segment of ultrasound image relating to the area of interest (ROI).

In fact, as it is known, the correlation between the position of each pixel in the ultrasound image and the ultrasound signal is carried out as a function of the time interval between the ultrasound pulse emission and the relative echo reception (reflected signal), since the signal reflected by the tissues positioned at greater depths uses more time to reach the probe after being reflected.

Therefore, regardless of the nature of the possible following processing, the segmentation of the "raw ultrasound signal corresponding to a determined ROI" occurs in the time domain, in order to isolate the raw ultrasound signal portion that, in the ultrasound image, originated a determined segment.

Yet, if not otherwise specified, it is intended that, according to what commonly known at the state of the art, the ultrasound probe used is a probe containing an array of piezoelectric transducers or CMUT configured to emit a plurality of ultrasound signals arranged side by side, so that to each signal a vertical line ("line of sight") of the ultrasound image corresponds and the assembly of the lines arranged side by side allows to make up the ultrasound image again. It is specified that, for brevity and clarity of description, the processing carried out on ultrasound signals will be described with reference to a single ultrasound signal. Obviously, even where it is not explicitly specified, it is clear that all the processing can be conveniently applied to a plurality of raw ultrasound signals, each one received by one of the piezoelectric transducers comprised in the ultrasound probe.

Yet, it is specified that with "point" of raw signal (radiofrequency ultrasound signal) it is intended the value of the raw signal in a single sampling point: in a 20 MHz sampling, as a way of purely example, 20000 points per millisecond of acquired signal are obtained.

It is also specified that all the processing described in the following is carried out by means of an echographic device provided with at least an ultrasound probe comprising at least an array of piezoelectric transducers or CMUT and suitable piloting means of said probe, with computing means for processing the signal configured to generate the signal to be sent by means of said probe and to analyze the signal received by said probe to obtain an ultrasound image, with user interaction means comprising a graphical interface and controlling means such as for example keyboards and/or pointing devices.

The device used comprises a HIFU ultrasound probe of the type known at the state of the art. Preferably, said probe comprises an array of piezoelectric transducers or CMUT configured to emit and receive ultrasound signals of frequency and intensity useful for the ultrasound imaging and an array of piezoelectric transducers configured to emit high intensity focused ultrasound signals. These devices, also as a whole, are known at the state of the art and commonly used in the ultrasound technique. However, it is specified that the ultrasound device used for carrying out the methods described in the following is configured to process the raw ultrasound signal (radiofrequency ultrasound signal) for determining an ultrasound image, but also to store the raw ultrasound signal in order to carry out following processing: it is a function not available in the ultrasound imaging devices available on the market.

As yet stated, a problem solved by the present invention is to provide a method for tumor mass treatment which, by means of a unique ultrasound probe, allows

(i) to individuate the tumor mass;
(ii) to define the area where the HIFU treatment is to be carried out;
(iii) to verify the correct positioning of the probe with respect to the tumor mass for the HIFU treatment;
(iv) to treat the tumor mass by means of HIFU;
(v) to verify the extension of the area actually treated.

The point (i) of tumor mass individuation can be carried out by means of an automatic tissue classification method, or by means of manual individuation by the doctor.

The definition of the area to be treated in point (ii) is proposed to the doctor as area identified as tumor, in addition to a buffer of possible further extension determinable by the doctor. It is clear that the definition of the area can be also defined by the doctor and highlighted on the ultrasound image by means of suitable commands provided on the echographic device.

As it is known, the effects of a HIFU treatment (positive if the treatment is applied to a tumor mass, negative if the treatment is applied to a healthy tissue) are correlated to the temperature reached by the tissues after the treatment; therefore, it is necessary to have a mean for measuring the temperature reached by the tissues treated during the treatment.

In fact, the key goal of all the ablative therapies is to reach and keep a temperature between 60 and 100°C in the whole volume of tissue to be treated to induce coagulative necrosis.

It has been said that in the treatment systems known at the state of the art, the temperature reached is not directly assessable in a reliable manner as a function of the treatment time, since the response to ultrasound insonification depends on the specific tissue considered and is variable from site to site of the human body, and in case of same site, from patient to patient.

In other words, it is not perfectly known, a priori, the entity of acoustic heating of each tissue as a response to a HIFU treatment.

The method according to the present invention allows instead to monitor the actual temperature distribution in ROI, during the whole duration of the HIFU treatment, in a non-invasive way and by means of exclusive ultrasound signal analysis.

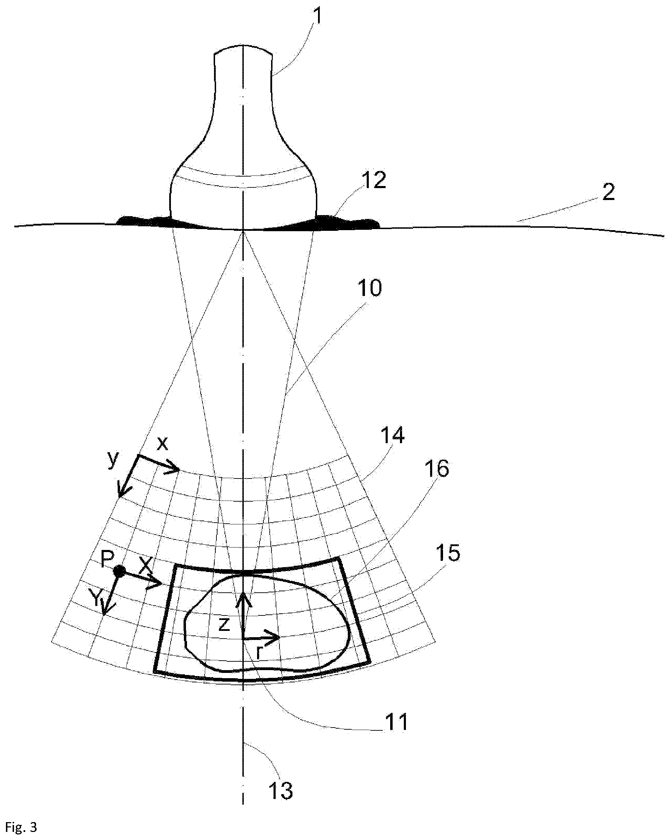

In figure 3 it is shown a scheme of a probe (1), resting on the skin of the patient (2), by means of interposition of coupling gel (12).

In figure 3, anatomical parts are intentionally not shown to mean that the method can be applied in various sites of the human body, according to the tissue or organ to be treated by HIFU.

It has to be specified that in the following, for simplicity, we will refer to the focal point (11) of the HIFU beam. With this term it is meant the centre of the focusing volume. The HIFU beam focusing volume can be schematized in a bidimensional ultrasound image by means of a focusing area. The focal point (11) indicated in the following is the centre of such focusing area.

As it is possible to observe, the probe (1) is configured:

(i) to emit and receive ultrasound pulses (200) in order to realize an ultrasound image (14) inside which an area of interest (15) is contained.
(ii) to emit a high intensity focused ultrasound beam (100), whose focus is positioned in a point (11) on the propagation axis (13) of the ultrasound beam.
(iii) to receive ultrasound signals reflected and/or emitted by the tissues of the patient after the HIFU treatment.

Conveniently, the probe can be configured to use a unique array of piezoelectric or CMUT transducers both to obtain the ultrasound image and to detect - clearly in discrete moments - the ultrasound signals reflected and/or emitted by the tissues of the patient after the HIFU treatment. As an alternative, the probe can comprise two arrays of transducers, one dedicated to the HIFU treatment and the other one to the acquisition of signals for the ultrasound imaging.

In other words, the device is configured to carry out cycles of:

- HIFU treatment by means of emission of a high intensity focused ultrasound beam (100),
- emission, during the break between emission of two focused ultrasound beams of the HIFU treatment, of a broadband ultrasound signal (200) and acquisition of the reflected ultrasound signal.

For each reflected raw ultrasound signal, the device is also configured to calculate the frequency spectrum (200s).

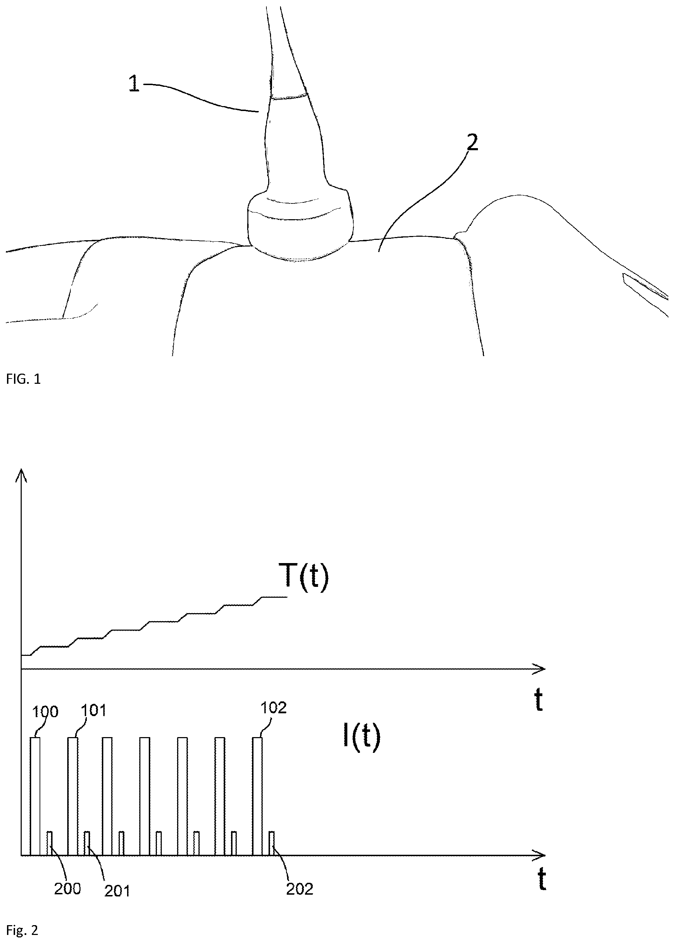

In figure 2, it is shown the time sequence of high intensity pulses (100, 101, 102) alternated with low intensity pulses (200, 201, 202).

It is to be noted how during each HIFU cycle the temperature increases, and so the following low intensity pulses are applied to a tissue with gradually increasing temperatures.

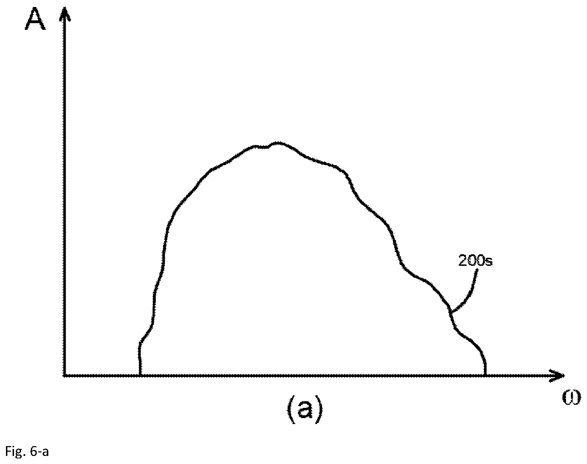

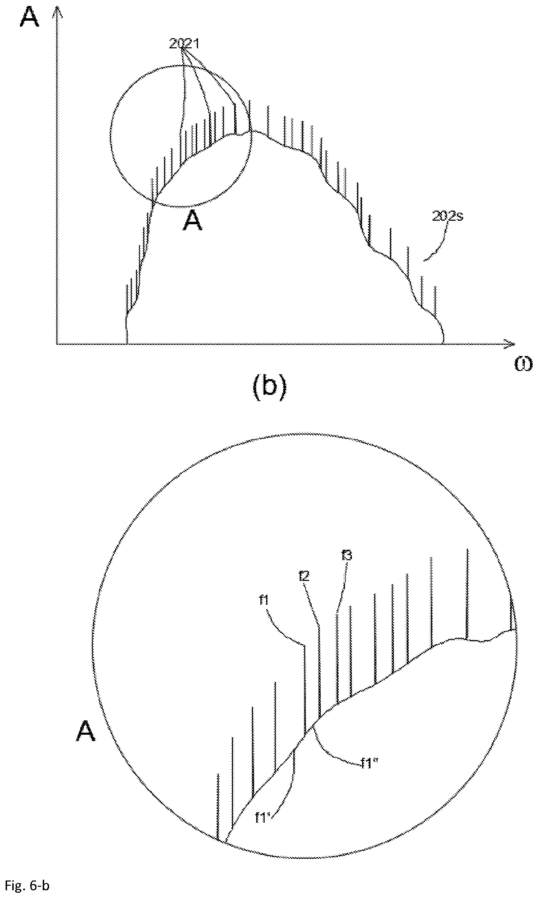

In figure 6, it is shown schematically the shape of the spectrum of the reflected signal in response to low intensity pulses, for temperatures with no localized boiling/cavitation phenomena (200s) and for temperatures at which localized boiling/cavitation phenomena (202s) are in progress.

Figure 3 schematically shows:

a) an area which has to undergo a treatment by means of HIFU (16);
b) an anatomical repere point (P), identified inside the ultrasound image.

Figure 3 also shows two reference systems:

c) a first cartesian reference systems (x, y), where the coordinate x identifies the propagation line associated to the specific piezoelectric or CMUT transducer of the array of piezoelectric or CMUT transducers contained inside the probe (1) and the coordinate y identifies the depth at which the point is positioned;
d) a polar coordinate system (r, z) originating in the focal point (11) of the HIFU ultrasound beam and axis z coincident with the emissions axis of the ultrasound pulses from the probe (1).

Therefore, in the ultrasound image plane it is possible to define a second coordinate system (X, Y) originated in the repere point P. This second coordinate system (X, Y) is fixed to the body of the patient, while the first two systems are integral to the probe (1). It is clear that each point of the ROI (15) can be identified in each of the three mentioned coordinate systems, and that by means of coordinate transformations with formulae already known at the state of the art, each point can be identified in each of the three mentioned coordinate systems.

After defining the coordinate systems, it is possible to determine firstly a method for monitoring the temperature distribution after the HIFU treatment, assuming that the ultrasound probe (1) is still in a static position.

Therefore, it is assumed that being a starting temperature distribution in ROI assigned, the time temperature variation in each point of the ROI after HIFU treatment can be expressed by the heating diffusion equation:

$$\frac{\partial T}{\partial t} = K \cdot \nabla^2 T + Q * I_0(r, z)$$

$$(1)$$

Wherein:

K is the thermal diffusivity [m2/sec];

$I_0(r,z)$ is the normalized profile of the spatial distribution of the acoustic signal intensity, i.e. a matrix of values between 0 and 1 which, in the reference system (r, z), defines the spatial distribution of the intensity of the signal emitted by the probe;

Q is the actual acoustic heating rate, and it is a scalar amount - expressed in °C/s - which links the emission of the probe to the actual tissue heating in the specific case.

While $I_0(r,z)$ is a feature of the ultrasound probe, and so it is known once the used ultrasound probe is characterized, Q depends on a plurality of specific parameters, such for example the tissue absorbing rate and the tissue attenuation rate, which are variable from patient to patient and, for each patient, inside each site, and so it is an unknown value.

It is specified that the possibility to determine correctly and in real time the value of the actual rate of acoustic heating Q for the specific patient can allow to calculate by means of equation (1) the temperature spatial and time distribution in tissues interested by the treatment, to view it in real time by means of the graphical interface of the sonographer and to allow the operator to carry out the treatment to avoid exceeding temperature thresholds that are critical for specific tissues close to the ones interested by the treatment.

The present invention allows to determine in real time and by using exclusively the just described ultrasound probe the acoustic heating rate value Q for the specific tissue object of treatment of each patient, and, so, to determine the temperature spatial and time distribution.

In order to obtain the temperature spatial distribution, the device according to the present invention is configured to carry out the following method:

a) identifying, inside an ultrasound image (14), a region of interest (15) inside which an area to be treated (16) is provided,

b) assigning a starting temperature distribution, by means of which a temperature value is assigned to each point of the ROI. Preferably, said starting temperature distribution is constant and equal to the patient body temperature, so typically equal to 37°C,

c) by keeping the probe (1) still in a static position, emitting a HIFU beam (100) focused on a focal point (11) contained inside said ROI, for a predetermined time interval, and subsequently a broadband ultrasound pulse (200), and detecting the ultrasound signal reflected and/or emitted by tissues in the treatment step. Preferably but not limitingly, said predetermined time interval is between 0.5 s and 1 s,

d) carrying out the frequency transform of said reflected ultrasound signal in response to said broadband ultrasound pulse (200), in order to obtain a reference frequency spectrum (200s),

e) repeating steps c) and d) iteratively, thus obtaining a frequency spectrum for each interaction,

f) assuming that the temperature at the focus (11) is equal to a predetermined temperature and function of the tissue under treatment when the frequency spectrum (202s) detected in response to a broadband ultrasound pulse (202) comprises a plurality of peaks (2021) not provided in the reference frequency spectrum (200s).

Preferably, said predetermined temperature is equal to 100°C in case of aqueous tissues. Said temperature can be possibly determined by tests on tissue portions (phantom), for which it is possible to measure the temperature directly, during execution.

g) determining the actual acoustic heating rate Q as a function of the irradiated signal and the thermal diffusivity intensity.

Preferably, the actual acoustic heating rate is calculated by solving equation (1) iteratively, in order to obtain the Q value;

h) using the actual acoustic heating rate value (Q) determined in point g) to calculate, by starting from temperature spatial distribution assigned in point c) and by means of heating diffusion equation, the temperature spatial distribution at the end of each time interval during which the tissue was subjected to HIFU in steps d) to f), thus obtaining the actual temperature distribution.

In this way, it was obtained both the determination of an actual heating rate value Q specific for tissues under treatment and a starting temperature distribution for the next treatment step.

It is specified that in a first embodiment said peaks (2021) can be individuated by carrying out the next steps for each iteration:

f.1) subtraction of the reference spectrum (200s) from the spectrum (202s) obtained after each HIFU treatment,

f.2) individuation of all the frequencies (f1, f2, f3), for which the difference between the two spectra (202s, 200s) is higher than a predetermined threshold, for example 5 dB,

f.3) classification of a frequency (f1) individuated as "peak" in the previous step, if at least a frequency lower (f1') than it and at least a frequency higher (f1") than it exists, for which said difference between the spectra is lower than a second predetermined threshold, for example 3 **dB.**

Moreover, preferably, in order to classify a frequency (f1) as peak, it is to be verified that the difference or the ratio between said lower (f1') and higher (f1") frequencies is lower than a predetermined amount, thus introducing a test condition of the peak amplitude.

It is to be specified that the temperature of 100°C at a significant change of the spectrum shape is justified by the fact that this value is a good assessment of the temperature at which localized boiling/cavitation phenomena can occur.

However, the whole operation is carried out with the device still and with the HIFU beam constantly focused on a unique point inside the ROI.

This means that only a very little region (ideally a point) of tissue inside the ROI, preferably chosen at the geometric center of gravity of the same, reaches the temperature of 100°C, while all the volume surrounding it reaches lower temperatures, thus guaranteeing the safety of treatment.

The device is also configured to calculate a plurality of parameters of shape of the frequency spectrum relating to the raw ultrasound signal reflected in response to the broadband ultrasound signal emitted during each break between emission of two focused ultrasound beams of HIFU treatment, and in particular of the portion of raw signal corresponding to a propagation path of the ultrasound signal comprising the focal point.

After determining the temperature time distribution, it is therefore possible to associate the respective focal point temperature to each calculated frequency spectrum (200s, 202s).

Therefore, the HIFU treatment can continue in other points localized inside the region to be treated.

In this case, the device is configured to measure the temperature at the focal point (1) relative to the new position of the probe:

- by calculating the frequency spectrum of the raw ultrasound signal reflected in response to the broadband ultrasound signal emitted during each break between the emission of two focused ultrasound beams of the HIFU treatment and, so,
- by calculating the correlation coefficient with each frequency spectrum associated to each temperature value.

As temperature value at the focus, it is assumed the temperature value for whose spectrum said correlation coefficient is higher.

In another embodiment said correlation coefficients are provided in decreasing order, and as temperature value it is assumed the average of temperature values associated to the highest correlation coefficients, for example to the 5 highest correlation coefficients.

It is specified that the condition verified in point f) needs to verify if boiling is observed at the focus (11). Since the treated tissues are aqueous media, in the present method it is in fact assumed that boiling starts at about 100°C. The presence of boiling causes not only that the tissue reflects the HIFU ultrasound pulses according to its own features, but that it has also an ultrasound emission of its own due to pulses emitted by water vapour bubbles forming, which can be detected by means of the frequency spectrum analysis. It is clear that the focus has to be inside the area to be treated, to avoid damaging healthy tissues.

Moreover, it is specified that for solving equation (1), in steps i) and j) a literature value for the K parameter can be conveniently used, or that the same can be determined directly by suitable tissue-mimicking phantoms.

Without this being limiting for the aims of the invention, the K value used is preferably between 1,1E-07 m2/s and 1,6E-07 m2/s.

Yet, it is specified that the iterative solution of equation (1) at point i) is carried out assuming that boiling occurs in primis at the focus.

Equation (1) is solved with an attempt value for Q, by calculating the temperature distribution for all the time intervals between moment t = 0 of treatment start, for which the temperature starting distribution was assigned, up to moment t = $t_{boil}$ in which boiling was detected by means of the frequency spectrum analysis. The Q value is then modified depending on the fact that the temperature value calculated at the focus for t = $t_{boil}$ is higher or lower than 100°C, and the procedure is repeated up to when the calculated value differs from 100°C by an amount lower than a predetermined threshold, for example 1°C.

As it is shown in figure (4), the indication relating to the calculated temperature distribution can be reproduced graphically by means of a series of isothermal curves (17), which, graphically overlapped with respect to the extension of the area to be treated, provide an immediate and efficient feedback to the operator.

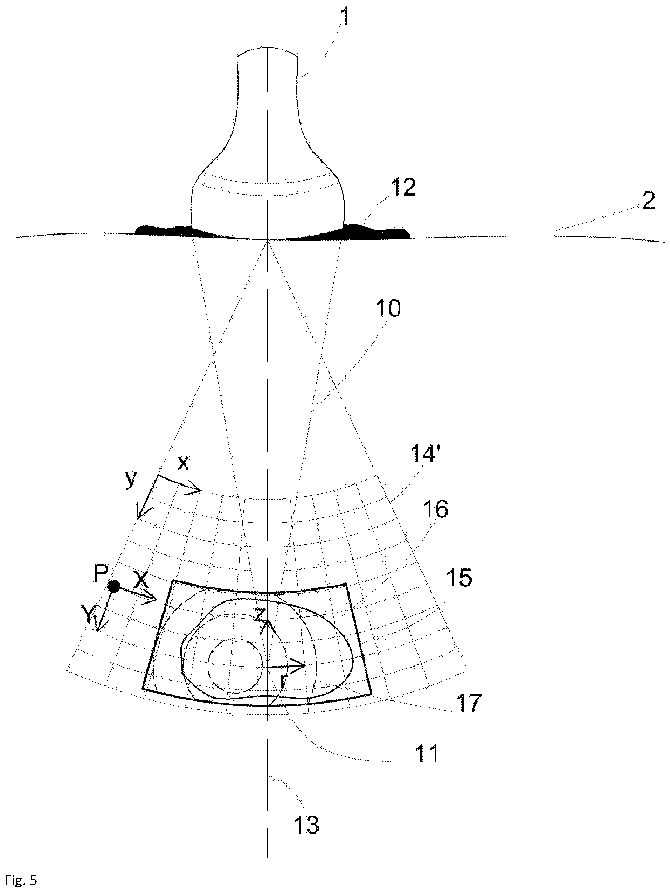

Moreover, the method proposed allows to verify also the actual temperature distribution inside the treated area, also in case of probe displacement. As it is shown in figure 5, in fact, after the displacement of the probe (1), the position of the focus (11) changes inside the area to be treated (16).

However, the identification on the new ultrasound image of the new position of the repere point (P) allows to correctly position the temperature spatial distribution values calculated in the previous probe position, since their coordinates (X, Y) in the coordinates system originated in the repere point are not changed.

Therefore, in the method according to the present invention, after each updating of temperature spatial distribution, conveniently carried out in the system (x, y) integral to the ultrasound probe, said temperature spatial distribution is transformed in the system (X, Y) originated in the repere point and integral thereto.

After probe displacement, the inverted operation will be carried out by transforming the temperature spatial distribution from the system integral to the repere point to the system integral to the ultrasound probe again.

## Claims

1. Ultrasound device configured to carry out a HIFU treatment and to detect in real time during the HIFU treatment the temperature distribution in the area of treatment, said device comprising:

   - an ultrasound probe comprising at least an array of piezoelectric or CMUT transducers;
   - piloting means of said ultrasound probe;
   - computing means configured to receive and store said raw ultrasound signals reflected by said tissues and acquired by each of said piezoelectric or CMUT transducers, to process said reflected raw ultrasound signals in order to generate an ultrasound image, as well as to carry out other processing on said raw ultrasound signals reflected by said tissues,

   wherein computer programs are loaded on said computing means, configured to carry out a method for determining the actual acoustic heating rate of tissues, comprising the following steps:

   a) identifying, inside an ultrasound image (14), a region of interest (15) inside which an area to be treated (16) is provided,
   b) assigning a starting temperature distribution, by means of which a temperature value is assigned to each point of ROI,
   c) emitting a high intensity ultrasound beam (100) focused on a focal point (11) contained in said ROI for a predetermined time interval, and subsequently a broadband ultrasound pulse (200), and detecting the ultrasound signal reflected and/or emitted by the tissues under treatment said method further comprises the following steps
   d) carrying out the frequency transform of said reflected ultrasound signal in response to said broadband ultrasound pulse (200), in order to obtain a reference frequency spectrum (200s),
   e) repeating steps c) and d) iteratively, thus obtaining a frequency spectrum for each iteration,
   f) assuming that the temperature at the focus (11) is equal to a predetermined temperature and function of the tissue under treatment when the frequency spectrum (202s) detected in response to a broadband ultrasound pulse (202) comprises a plurality of peaks (2021) not provided in the reference frequency spectrum (200s),
   g) determining the actual acoustic heating rate Q as a function of said predetermined temperature and of the intensity of said high intensity ultrasound beam (100) wherein the actual acoustic heating rate is calculated by solving iteratively the equation of heating diffusion, by assuming as starting condition said temperature distribution assigned at point b), wherein

said device is configured to associate the respective focal point temperature to each frequency spectrum (200s, 202s) calculated after the emission of each high intensity ultrasound beam (100), and wherein the device is configured to measure the temperature distribution of tissues undergoing the HIFU treatment at the focal point (11) relative to a new position of said probe:

- by calculating the frequency spectrum of the raw ultrasound signal reflected in response to the broadband ultrasound signal emitted during each break between the emission of two focused ultrasound beams of the HIFU treatment,
- by calculating the correlation coefficient with each frequency spectra associated to each temperature value,
- by assuming the temperature value for whose spectrum said correlation coefficient is the highest as temperature value of tissues at said focal point (11).

2. Device according to claim 1, **characterized in that** said device is configured for providing said correlation coefficients in decreasing order, and for assuming the temperature values average associated to the highest correlation coefficients, for example to the 5 highest correlation coefficients, as temperature value at said focal point (11).

3. Device according to any one of the preceding claims, **characterized in that** said predetermined temperature is equal to 100°C.

4. Device according to claim 1, **characterized in that** said temperature is determined by tests on tissue portions, for which it is possible to measure the temperature directly, during execution.

5. Device according to any one of the preceding claims, **characterized in that** said peaks (2021) are individuated by carrying out the next steps for each iteration:

f.1) subtraction of the reference spectrum (200s) from the spectrum (202s) obtained after each HIFU treatment,
f.2) individuation of all the frequencies (f1, f2, f3), for which the difference between the two spectra (202s, 200s) is higher than a predetermined threshold,
f.3) classification of a frequency (f1) individuated as "peak" in the previous step, if at least a frequency lower (f1') than it and at least a frequency higher (f1") than it exists, for which said difference between the spectra is lower than a second predetermined threshold.

6. Device according to claim 5, **characterized in that** it is configured to verify further, before classifying a frequency (f1) as peak, that the difference or the ratio between said lower (f1') and higher (f1") frequencies is lower than a predetermined amount.

7. Device according to any one of the preceding claims, configured to show the temperature distribution of tissues by means of said graphical user interface, by means of a series of isothermal curves (17) graphically overlapped on an ultrasound image with respect to the extension of the area to be treated.

8. Device according to any one of the preceding claims, further configured:

- to recognize inside said ultrasound image (14) an anatomical repere point (P), thus identifying its position,
- to define a reference system (X, Y) integral to said anatomical repere point,
- to assign a value of coordinates in said reference system (X, Y) integral to said repere point (P) to each point of said ROI for which said temperature distribution was defined,
- to displace said probe (1) a in a new position in which said focal point is still contained inside said area to be treated (16) and said repere point (P) is visible in the new ultrasound image (14') obtained by means of said probe (1) in the new position,
- to individuate on said new ultrasound image (14') the position of said ROI (15) and of said repere point (P),
- to assign a coordinate value in the coordinate system (x, y) integral to the probe to each point of said ROI for which said temperature distribution was defined,
- to view said temperature distribution overlapped to said new ultrasound image.

**Patentansprüche**

1. Ultraschallvorrichtung, konfiguriert zur Durchführung einer HIFU-Behandlung und zur Echtzeit-Erfassung der Temperaturverteilung im zu behandelnden Bereich bei der HIFU-Behandlung, wobei die Vorrichtung umfasst:

- eine Ultraschallsonde mit mindestens einer Mehrzahl von piezoelektrischen Wandlern oder CMUTs;
- Mittel zur Steuerung der Ultraschallsonde;
- Rechenmittel zum Empfangen und Speichern der vom Gewebe reflektierten und von jedem der piezoelektrischen Wandler oder CMUTs erfassten Roh-Ultraschallsignale, zur Verarbeitung von solchen reflektierten Roh-Ultraschallsignalen zur Erzeugung eines Ultraschallbildes sowie zur weiteren Verarbeitung der vom Gewebe reflektierten Roh-Ultraschallsignale,

wobei auf diese Rechenmittel werden Computerprogramme geladen, die konfiguriert sind, um ein Verfahren zur Bestimmung der tatsächlichen akustischen Erwärmungsrate des Gewebes zu implementieren, mit den folgenden Schritten:

a) Identifizierung eines Zielbereichs (15) mit einem zu behandelnden Bereich (16) in einem Ultraschallbild (14);

b) Zuweisung einer anfänglichen Temperaturverteilung, die die Zuordnung eines Temperaturwerts zu jedem ROI-Punkt ermöglicht;

c) Aussenden eines hochintensiven Ultraschallstrahls (100), der für ein vorgegebenes Zeitintervall auf einen Brennpunkt (11) im ROI fokussiert ist, anschließend eines Breitband-Ultraschallimpulses (200) und Erfassen des vom behandelten Gewebe reflektierten und/oder emittierten Ultraschallsignals,

wobei das Verfahren ferner umfasst die folgenden Schritte:

d) Durchführen der Frequenztransformation des reflektierten Ultraschallsignals als Reaktion auf den Breitband-Ultraschallimpuls (200), um ein Referenzfrequenzspektrum (200s) zu erhalten;

e) iteratives Wiederholen der Schritte c) und d), wodurch bei jeder Iteration ein Frequenzspektrum erhalten wird;

f) Annehmen, dass die Temperatur (beim Fokus 11) einer vorgegebenen Temperatur entspricht und vom behandelten Gewebe abhängt, wenn das als Reaktion auf einen Breitband-Ultraschallimpuls (202) erfasste Frequenzspektrum (202s) mehrere Peaks (2021) aufweist, die im Referenzfrequenzspektrum (200s) nicht vorhanden sind;

g) Bestimmen der tatsächlichen akustischen Heizungsrate Q als Funktion der vorgegebenen Temperatur und der Intensität des hochintensiven Ultraschallstrahls (100), wobei die tatsächliche akustische Heizungsrate durch iteratives Lösen der Heizdiffusionsgleichung berechnet wird, wobei als Ausgangsbedingung die in Punkt b) angegebene Temperaturverteilung angenommen wird,

wobei die Vorrichtung so konfiguriert ist, dass sie die Temperatur des jeweiligen Brennpunkts jedem Frequenzspektrum (200s, 202s) zuordnet, das nach der Emission jedes hochintensiven Ultraschallstrahls (100) berechnet wurde, und

wobei die Vorrichtung so konfiguriert ist, dass sie die Temperaturverteilung des der HIFU-Behandlung unterzogenen Gewebes am Brennpunkt (11) relativ zu einer neuen Position der Sonde misst:

- durch Berechnung des Frequenzspektrums des Roh-Ultraschallsignals, das als Reaktion auf das während jeder Unterbrechung zwischen der Emission zweier fokussierter Ultraschallstrahlen der HIFU-Behandlung emittierte Breitband-Ultraschallsignal reflektiert wird;

- durch Berechnung des Korrelationskoeffizienten mit jedem Frequenzspektrum, das jedem Temperaturwert zugeordnet ist;

- mit der Annahme, dass der Temperaturwert für solche Spektren mit dem höchsten Korrelationskoeffizienten als Gewebetemperaturwert am Brennpunkt (11) betrachtet wird.

2. Vorrichtung nach Anspruch 1, **dadurch gekennzeichnet, dass** die Vorrichtung die Korrelationskoeffizienten in absteigender Reihenfolge bereitstellt und den Durchschnitt der Temperaturwerte mit den höchsten Korrelationskoeffizienten, beispielsweise den 5 höchsten Korrelationskoeffizienten, als Temperaturwert im Brennpunkt (11) berücksichtigt.

3. Vorrichtung nach einem der vorhergehenden Ansprüche, **dadurch gekennzeichnet, dass** die vorgegebene Temperatur 100°C beträgt.

4. Vorrichtung nach Anspruch 1, **dadurch gekennzeichnet, dass** die Temperatur durch Tests an Gewebepartien ermittelt wird, deren Temperatur während der Durchführung direkt gemessen werden kann.

5. Vorrichtung nach einem der vorhergehenden Ansprüche, **dadurch gekennzeichnet, dass** die Peaks (2021) durch die Durchführung der folgenden Schritte für jede Iteration individualisiert werden:

f.1) Subtraktion des Referenzspektrums (200s) vom nach jeder HIFU-Behandlung erhaltenen Spektrum (202s);
f.2) Identifizierung aller Frequenzen (f1, f2, f3), bei denen die Differenz zwischen den beiden Spektren (202s, 200s) größer als ein vorgegebener Schwellenwert ist;
f.3) Klassifizierung einer im vorherigen Schritt als "Peak" identifizierten Frequenz (f1), sofern mindestens eine niedrigere Frequenz (f1') und mindestens eine höhere Frequenz (f1") vorhanden sind, bei denen die Differenz zwischen den Spektren kleiner als ein zweiter vorgegebener Schwellenwert ist.

6. Vorrichtung nach Anspruch 5, **dadurch gekennzeichnet, dass** sie so konfiguriert ist, dass sie vor der Klassifizierung einer Frequenz (f1) als Peak überprüft, ob die Differenz oder das Verhältnis zwischen den niedrigeren (f1') und höheren (f1") Frequenzen kleiner als ein vorgegebener Wert ist.

7. Vorrichtung nach einem der vorhergehenden Ansprüche, konfiguriert zur Anzeige der Gewebetemperaturverteilung über die grafische Benutzeroberfläche mittels einer Reihe von Isothermenkurven (17), die grafisch über ein Ultraschallbild im Verhältnis zur Ausdehnung des zu behandelnden Bereichs gelegt werden.

8. Vorrichtung nach einem der vorhergehenden Ansprüche, ferner konfiguriert für:

- Erkennung einer anatomischen Landmarke (P) im Ultraschallbild (14) und damit Identifizierung ihrer Position;
- Definition eines Referenzsystems (X, Y), das mit der anatomischen Landmarke integriert ist;
- Zuweisung eines Koordinatenwerts im Referenzsystem (X, Y), das mit der Landmarke (P) integriert ist, zu jedem Punkt des ROI, für den die Temperaturverteilung definiert wurde;
- Bewegung der Sonde (1) an eine neue Position, in der sich der Fokuspunkt noch innerhalb des zu behandelnden Bereichs (16) befindet und die Landmarke (P) auf dem neuen Ultraschallbild (14'), das mit der Sonde (1) an der neuen Position aufgenommen wurde, sichtbar ist;
- Identifizierung der Position der ROI (15) und der Landmarke (P) auf dem neuen Ultraschallbild (14');
- Zuweisung eines Koordinatenwerts im in die Sonde integrierten Koordinatensystem (x, y) zu jedem Punkt der ROI, für den die Temperaturverteilung definiert wurde;
- Visualisierung der Temperaturverteilung überlagert auf dem neuen Ultraschallbild.

**Revendications**

1. Dispositif à ultrasons configuré pour réaliser un traitement HIFU et détecter en temps réel, pendant le traitement HIFU, la répartition de la température dans la zone à traiter, ledit dispositif comprenant :

- une sonde à ultrasons comprenant au moins un réseau de transducteurs piézoélectriques ou CMUT ;
- des moyens de pilotage de ladite sonde à ultrasons ;
- des moyens de calcul configurés pour recevoir et stocker les signaux ultrasonores bruts réfléchis par les tissus et acquis par chacun des transducteurs piézoélectriques ou CMUT, pour traiter lesdits signaux ultrasonores bruts réfléchis afin de générer une image ultrasonore, ainsi que pour effectuer d'autres traitements sur ces signaux ultrasonores bruts réfléchis par lesdits tissus,
dans lequel des programmes informatiques sont chargés sur ces moyens de calcul, configurés pour mettre en œuvre une méthode de détermination de la vitesse de chauffage acoustique réelle des tissus, comprenant les étapes suivantes :

a) identification, dans une image ultrasonore (14), d'une région d'intérêt (15) contenant une zone à traiter (16) ;
b) attribution d'une distribution de température initiale, par laquelle une valeur de température est attribuée à chaque point de ROI ;
c) émission d'un faisceau ultrasonore de haute intensité (100) focalisé sur un point focal (11) contenu dans ledit ROI pendant un intervalle de temps prédéterminé, puis d'une impulsion ultrasonore à large bande (200), et détection du signal ultrasonore réfléchi et/ou émis par les tissus traités,

ladite méthode comprenant en outre les étapes suivantes

d) réaliser la transformée de fréquence dudit signal ultrasonore réfléchi en réponse à ladite impulsion ultrasonore à large bande (200), afin d'obtenir un spectre de fréquences de référence (200s),

e) répéter les étapes c) et d) de manière itérative, obtenant ainsi un spectre de fréquences à chaque itération,

f) supposer que la température du focus (en 11) est égale à une température prédéterminée et dépendante du tissu traité lorsque le spectre de fréquences (202s) détecté en réponse à une impulsion ultrasonore à large bande (202) comprend une pluralité de pics (2021) non présents dans le spectre de fréquences de référence (200s),

g) déterminer la vitesse de chauffage acoustique réelle Q en fonction de ladite température prédéterminée et de l'intensité dudit faisceau ultrasonore de haute intensité (100), la vitesse de chauffage acoustique réelle étant calculée en résolvant de manière itérative l'équation de diffusion du chauffage, en supposant comme condition de départ ladite distribution de température attribuée au point b),

dans lequel le dispositif est configuré pour associer la température du point focal correspondant à chaque spectre de fréquences (200s, 202s) calculé après l'émission de chaque faisceau ultrasonore de haute intensité (100), et dans lequel le dispositif est configuré pour mesurer la distribution de température des tissus soumis au traitement HIFU au point focal (11) par rapport à une nouvelle position de la sonde :

- en calculant le spectre de fréquences du signal ultrasonore brut réfléchi en réponse au signal ultrasonore à large bande émis lors de chaque interruption entre l'émission de deux faisceaux ultrasonores focalisés du traitement HIFU ;

- en calculant le coefficient de corrélation avec chaque spectre de fréquences associé à chaque valeur de température ;

- en considérant comme valeur de température des tissus au point focal (11), la valeur de température pour le spectre de laquelle le coefficient de corrélation est le plus élevé.

2. Dispositif selon la revendication 1, **caractérisé en ce que** ledit dispositif est configuré pour fournir lesdits coefficients de corrélation par ordre décroissant et pour considérer la moyenne des valeurs de température associées aux coefficients de corrélation les plus élevés, par exemple aux 5 coefficients de corrélation les plus élevés, comme valeur de température au point focal (11).

3. Dispositif selon l'une quelconque des revendications précédentes, **caractérisé en ce que** ladite température prédéterminée est égale à 100°C.

4. Dispositif selon la revendication 1, **caractérisé en ce que** ladite température est déterminée par des tests sur des portions de tissu, pour lesquels il est possible de mesurer la température directement, pendant l'exécution.

5. Dispositif selon l'une quelconque des revendications précédentes, **caractérisé en ce que** lesdits pics (2021) sont individualisés en effectuant les étapes suivantes pour chaque itération :

f.1) soustraction du spectre de référence (200s) du spectre (202s) obtenu après chaque traitement HIFU ;

f.2) individuation de toutes les fréquences (f1, f2, f3) pour lesquelles la différence entre les deux spectres (202s, 200s) est supérieure à un seuil prédéterminé ;

f.3) classification d'une fréquence (f1) individualisée comme « pic » à l'étape précédente, s'il existe au moins une fréquence inférieure (f1') à celle-ci et au moins une fréquence supérieure (f1") à celle-ci, pour lesquelles ladite différence entre les spectres est inférieure à un deuxième seuil prédéterminé.

6. Dispositif selon la revendication 5, **caractérisé en ce qu'**il est configuré pour vérifier en outre, avant de classer une fréquence (f1) comme un pic, que la différence ou le rapport entre lesdites fréquences inférieure (f1') et supérieure (f1") est inférieure à une valeur prédéterminée.

7. Dispositif selon l'une quelconque des revendications précédentes, configuré pour afficher la distribution de la température des tissus au moyen de ladite interface utilisateur graphique, au moyen d'une série de courbes isothermes (17) superposées graphiquement sur une image ultrasonore par rapport à l'étendue de la zone à traiter.

8. Dispositif selon l'une quelconque des revendications précédentes, configuré en outre pour :

- reconnaître un point de repère anatomique (P) dans ladite image ultrasonore (14) et ainsi identifier sa position ;
- définir un système de référence (X, Y) intégré audit point de repère anatomique ;
- attribuer une valeur de coordonnées dans ledit système de référence (X, Y) intégrée audit point de repère (P) à chaque point dudit ROI pour lequel ladite distribution de température a été définie ;
- déplacer ladite sonde (1) vers une nouvelle position dans laquelle ledit point focal est toujours contenu dans ladite zone à traiter (16) et ledit point de repère (P) est visible sur la nouvelle image ultrasonore (14') obtenue au moyen de ladite sonde (1) dans la nouvelle position ;
- identifier sur ladite nouvelle image ultrasonore (14') la position dudit ROI (15) et dudit point de repère (P);
- attribuer une valeur de coordonnées dans le système de coordonnées (x, y) intégré à la sonde à chaque point dudit ROI pour laquelle ladite distribution de température a été définie,
- visualiser ladite distribution de température superposée à ladite nouvelle image ultrasonore.

FIG. 1

Fig. 2

Fig. 3

Fig. 4

Fig. 5

(a)

Fig. 6-a

(b)

Fig. 6-b

**REFERENCES CITED IN THE DESCRIPTION**

**Patent documents cited in the description**

- US 2015005634 A
- US 2020237234 A
- US 2019142513 A
- US 2019262074 A
- WO 2019173138 A
- US 8016757 B